**Europäisches Patentamt**

**European Patent Office**

**Office européen des brevets**

(19)

(11) Publication number : **0 070 077 B2**

# (12) NEW EUROPEAN PATENT SPECIFICATION

(45) Date of publication of the new patent specification : **04.11.92 Bulletin 92/45**

(51) Int. Cl.⁵ : **C11D 1/83,** C11D 3/04

(21) Application number : **82200871.0**

(22) Date of filing : **12.07.82**

(54) **Light-duty detergent granule composition.**

The file contains technical information submitted after the application was filed and not included in this specification

(30) Priority : **26.04.82 US 371696**
**13.07.81 US 282976**

(43) Date of publication of application :
**19.01.83 Bulletin 83/03**

(45) Publication of the grant of the patent :
**21.01.87 Bulletin 87/04**

(45) Mention of the opposition decision :
**04.11.92 Bulletin 92/45**

(84) Designated Contracting States :
**AT BE CH DE FR GB IT LI NL**

(56) References cited :
**DE-B- 593 422**
**FR-A- 2 093 790**
**US-A- 3 721 633**
**US-A- 3 839 318**

(56) References cited :
**US-A- 4 154 706**
**US-A- 4 240 921**
**Fette, Seifen, Anstrichmittel, Die Ernährungsindustrie 68, 1966, pp. 551-556**
**JAOCS 47, 1970, pp. 162-167**

(73) Proprietor : **THE PROCTER & GAMBLE COMPANY**
**301 East Sixth Street**
**Cincinnati Ohio 45202 (US)**

(72) Inventor : **Llenado, Ramon Aquillon**
**8090 Pepper Pike**
**West Chester, OH 45069 (US)**
Inventor : **Mao, Mark Hsiang K.**
**4114 Fox Hollow Dr.**
**Cincinnati, OH 45241 (US)**
Inventor : **Nicholson, Denzel Allan**
**472 Lakeridge Dr.**
**Cincinnati, OH 45231 (US)**

(74) Representative : **Canonici, Jean-Jacques et al**
**Procter & Gamble European Technical Center**
**N.V. Temselaan 100**
**B-1853 Strombeek-Bever (BE)**

EP 0 070 077 B2

## Description

This invention relates to detergent granules which provide controllable aqueous foams. Such compositions can be used in any situation where foams are desirable, including the laundry; personal cleaning products and dishwashing.

Alkylpolyglucosides which are surfactants have been disclosed in U.S. patents 3,598,865, 3,721,633 and 3,772,269. These patents also disclose processes for making alkylpolyglucoside surfactants and built liquid detergent compositions containing these surfactants. U.S. patent 3,219,656 discloses alkylmonoglucosides and suggests their utility as foam stabilisers for other surfactants. Various polyglucoside surfactant structures and processes for making them are disclosed in U.S. patents 2,974,134, 3,640,998, 3,839,318, 3,314,936, 3,346,558, 4,011,389 and 4,223,129.

Granulate detergent compositions containing an alkyl benzene sulfonate and a water-soluble inorganie soll au known from Fette - Seifen - Anstrichmittel - Die Ernähungsindustrin 68, 551-556 (1966)

Foaming compositions containing an alkylpolysaccharide surfactant, an anionic cosurfactant and possibly an auxiliary foam booster, are described in copending published EP-A-0070074, EP-A-0070075 and EP-A-0070076.

All percentages, parts and ratios used herein are by weight unless otherwise specified.

This invention relates to agglomerated light duty detergent granule compositions comprising

(1) from 5 to 60%, preferably from 10% to 20% of an alkylpolyglucoside having the formula $R^2O(C_nH_{2n}O)_t(Z)_x$ wherein Z is derived from glucose, $R^2$ is a saturated hydrophobic alkyl group containing from 12 to 18, preferably from 12 to 14 carbon atoms; n is 2 or 3, preferably 2, t is from 0 to 10, preferably 0; and x is from 1.5 to 4, most preferably from 1.6 to 2.7, and including less than 10% unreacted fatty alcohol and less than 50% short chain alkylpolyglucoside;

(2) from 5% to 60% of an alkyl benzene sulfonate cosurfactant in which the alkyl group contains from 10 to 13 carbon atoms, said alkyl benzene sulfonate neutralised with one or more cationic moieties (M) to balance the formula;

(3) from 5% to 60%, preferably from 10% to 20% of an alkylpolyethoxylate sulfate cosurfactant in which the alkyl group contains from 10 to 16 carbon atoms and in which there are from 1 to 6 ethoxylate groups, said alkylpolyethoxylate sulfate neutralised with one or more cationic moieties M to complete the formula;

(4) from 5% to 80% of a water soluble inorganic salt selected from the group consisting of sodium and potassium sulfates, chlorides, carbonates, phosphates, and mixtures thereof,

made by agglomeration.

It has surprisingly been found that the cosurfactants interact with the alkylpolyglucoside surfactant to provide a relatively stable foam which is readily rinsed, and that the composition can be formulated as agglomerated granules.

The alkylpolyglucosides are those having an alkyl hydrophobic group containing from 12 to 18 carbon atoms, preferably from 12 to 16 carbon atoms, most preferably from 12 to 14 carbon atoms. The number x indicates the number of glucoside units in a particular alkylpolyglucoside surfactant. For a particular alkylpolyglucoside molecule x can only assume integral values. In any physical sample of alkylpolyglucoside surfactants there will in general be molecules having different x values. The physical sample can be characterised by the average value of x and this average value can assume non-integral values. In this specification the values of x are to be understood to be average values. The hydrophobic group ($R^2$) can be attached at the 2-, 3- or 4-positions rather than at the 1-position, (thus giving, e.g., a glucosyl as opposed to a glucoside). However, attachment through the 1-position, i.e., glucosides, is preferred. In the preferred product the additional glucoside units are predominately attached to the previous saccharide unit's 2-position. Attachment through the 3-, 4- and 6-positions can also occur.

Optionally and less desirably there can be a polyalkoxide chain joining the hydrophobic moiety ($R^2$) and the polyglucoside chain. The preferred alkoxide moiety is ethoxide.

Suitable alkylpolyglucosides are decyl, dodecyl, tetradecyl, hexadecyl and octadecyl, di-, tri-, tetra-, penta- and hexaglucosides.

The alkylmonoglucosides are relatively less soluble in water than the higher alkylpolyglucosides. When used in admixture with alkylpolyglucoside, the alkylmonoglucosides are solubilised to some extent. The use of alkylmonoglucosides in admixture with alkylpolyglucosides is a preferred mode of carrying out the invention. Suitable mixtures include coconut alkyl, di-, tri-, tetra- and pentaglucosides and tallow alkyl tetra-, penta- and hexaglucosides.

To prepare these compounds a long chain alcohol ($R^2OH$ can be reacted with glucose, in the presence of an acid catalyst to form the desired glucoside. Alternatively the alkylpolyglucosides can be prepared by a two step procedure in which a short chain alcohol ($C_{1-16}$) is reacted with glucose or a polyglucoside (x=2 to 4) to yield a short chain alkyl glucoside (x=1 to 4) which can in turn be reacted with a longer chain alcohol ($R^2OH$) to displace the short chain alcohol and obtain the desired alkylpolyglucoside. If this two step procedure is used, the short chain alkylglucoside content of the final alkylpolyglucoside material should be less than 50%, preferably less than 10%, more preferably less than 5%, most preferably 0% of the alkylpolyglucoside.

The amount of unreacted alcohol (the free fatty alcohol content) in the desired alkylpolyglucoside surfactant is preferably less than 2%, more preferably less than 0.5% by weight of the total of the alkylpolyglucoside plus unreacted alcohol. The amount of alkylmonoglucoside is 20% to 70%, preferably 30% to 60%, most preferably 30% to 50% by weight of the total of the alkylpolyglucoside. For some uses it is desirable to have the alkylmonoglucoside content less than 10%.

Through this specification, "alkylglucoside" is used to include alkylglycosides because the stereochemistry of the glucoside moiety is changed during the preparation reaction.

Anionic surfactants can be selected from the group consisting of sulfates, sulfonates, and mixtures thereof. The surfactants are neutralised with a cationic moiety or moieties selected from the group consisting of alkali metal, e.g., sodium or potassium, alkaline earth metal, e.g., calcium or magnesium, ammonium, substituted ammonium, including mono-, di-, or tri-, ethanolammonium cations. Mixtures of cations can be desirable. The anionic surfactants useful in the present invention all have detergent properties and are all water-soluble or dispersible in water.

One of the surfactants for use in this invention an alkylbenzene sulfonate. The alkyl group can be either saturated or unsaturated, branched or straight chain and is optionally substituted with a hydroxy group. Middle phenyl positions are generally preferred for volume of foaming in light soil conditions. However, in heavier soil conditions, phenyl attachment at the 1- or 2- position is preferred.

The preferred alkylbenzene sulfonates contain a straight alkyl chain containing from 10 to 13 carbon atoms, and the cation is sodium, potassium, ammonium, mono-, di- or triethanolammonium, calcium or magnesium and mixtures thereof. Magnesium is the preferred cationic moiety. These same cations are preferred for other anionic surfactants and ingredients. The magnesium alkylbenzene sulfonates where the phenyl group is attached near the middle of the alkyl chain are surprisingly better than the ones with the phenyl near the end of the chain when the polyglucoside chain averages greater than 3 glucoside units. Suitable alkylbenzene sulfonates include $C_{11}$ alkylbenzene sulfonates with low 2-phenyl content.

The alkylbenzene sulfonate surfactant is desirable in the foaming compositions of the invention since the foams produced therewith are exceptionally stable, have a large volume, rinse quickly, and do not have a "slippery" feel. These compositions are particularly desirable for industrial and commercial processes as discussed hereinafter. The volume of foam produced using the alkylbenzene sulfonate cosurfactant is larger than for any other cosurfactant.

The alkylpolyglucosides can be contaminated with less than 50% short chain alkylpolyglucosides and with less than 10%, preferably less than 2%, most preferably less than 0.5% unreacted fatty alcohol, and increase the sudsing ability of conventional sulfate detergent cosurfactants, especially alkyl sulfate and alkyl polyether sulfate cosurfactants having the formula $R^3O(C_nH_{2n}O)_tSO_3M$ wherein $R^3$ is an alkyl or hydroxyalkyl group containing from 8 to 18 carbon atoms, n is 2 or 3, t can vary from 0 to 30, and M is a cationic moiety as defined above, the cosurfactant being water soluble or dispersible.

Mixtures of alkylbenzene sulfonate and the alkylpolyglucoside surfactant can be used at levels of from 0.01% to 95%, in ratios of cosurfactant to alkylpolysaccharide of from 10:1 to 1:10, in water with agitation to provide foams. These foams are relatively stable and, if not disturbed, can exist for several days. Furthermore, the foam has structural integrity and does not spread out. The foams prepared using mixtures of alkylbenzene sulfonate and the alkylpolyglucoside are unique in that they do not have a "slippery" feel. all of the foams rinse quickly.

The unusual properties of the foams make the granular compositions valuable for use in laundry and dishwashing, where a good volume of stable suds and quick rinsability are desirable.

The compositions of this invention can utilise other compatible ingredients, including other surfactants, in addition to the mixture of surfactants herein disclosed. In detergent compositions the compositions can contain any of the well known ingredients including minor amounts of other surfactants, detergency builders, soil suspending agents, brighteners, abrasives, dyes, fabric conditioning agents, hair conditioning agents, hydrotopes, solvents, fillers, clays or perfumes. Suitable ingredients are disclosed in U.S. patents 4,166,039 - Wise, 4,157,978 - Llenado, 4,056,481 - Tate, 4,049,586 - Collier, 4,035,257 - Cherney, 4,019,998 - Benson et al, 4,000,080 - Bartolotta et al and 3,983,078 - Collins. Listings of suitable additional ingredients, including low levels of other surfactants can be found in U.S. patent 4,089,945, 3,987,161 and 3,962,418.

Of special interest are ingredients which modify the feel of aqueous solutions containing the foaming compositions of this invention. For example, raising the pH to above 8.5 by alkaline materials or incorporating the tertiary alcohols of EP-A-0049546. Such ingredients are desirable for some consumers since the solutions do not have the normal "soapy" feel associated with surfactant solutions.

The following non-limiting examples illustrate the foaming compositions of the present invention.

The relative volume of suds in ml is determined by the following test procedure.

100ml of the test solution at 46.1°C is placed in a 500ml graduated cylinder: the solution is agitated by repeated inversion of the graduated cylinder until the amount of suds in the cylinder does not increase with further agitation. Suds height is measured directly on the cylinder scale making allowance for the height of liquid remaining in the cylinder. The test solution is made by adding the test product to water having a hardness of 0.12 gram per liter (Ca/Mg=3/1).

Example 1

105 grams of sodium dodecylbenzene sulfonate are mixed with 350 grams of anhydrous sodium sulfate. After the mixture is ground into a fine powder, 70g of $C_{12-13}$ alkylpolyglucoside ($G_{2,2}$) (<2% free fatty alcohol) are then mixed in. the mixture is transferred into a fluid bed dryer operated at room temperature (e.g., Aeromatic Inc., Model STREA-1), then 100 grams of a 50% solution of said alkylpolyglucoside is sprayed on to the powder. 7.5 milliliters of a 1% polar blue solution are sprayed on to the powder and a small portion of perfume is then added. The resulting granule is dried in a vacuum oven at 30 in of Hg vacuum at 50°C for ten hours to remove excess water.

In a similar manner 60 grams of a 50% solution of said alkylpolyglucoside is sprayed on to 100 gram of Berkite and 50 grams of sodium dodecylbenzene sulfonate flakes are admixed with the product to give a light duty granule.

Known analytical techniques can be used to determine the structures of the alkylpolyglucoside surfactants herein; for example, to determine the glucosidic chain length, the amount of butyl glucoside, the free fatty alcohol content, and the level of unreacted polyglucoside. More specifically, gas or liquid chromatography can be used to determine the unreacted alcohol content and the unreacted polyglucoside content respectively. Proton nmr can be used to determine the average glucosidic chain length. the point of attachment of the hydrophilic portion of the molecule to the hydrophobic portion of the molecule can be determined by $^{13}$C nmr.

The alkylpolysaccharide surfactants are complex mixtures. Their components vary depending upon the nature of the starting materials and the reaction by which they are prepared. Analytical standards which are useful in calibrating instruments for analysing the components of a particular alkylpolyglucoside surfactant can be obtained from Calbiochem Behring Co. LaJolla, California. These standards include those for octylglucoside (Calbiochem #494559), decylglucoside (Calbiochem #252715), dodecylmaltoside (Calbiochem #3243555).

## Claims

1. A light-duty detergent granule composition comprising
   (1) from 5% to 60% by weight of an alkyl benzene sulfonate cosurfactant in which the alkyl group contains from 10 to 13 carbon atoms, said alkyl benzene sulfonate neutralised with one or more cationic moieties;
   (2) from 5% to 60% by weight of an alkylpolyethoxylate sulfate cosurfactant in which the alkyl group contains from 10 to 16 carbon atoms and there are from 1 to 6 ethoxylate groups, said alkylpolyethoxylate sulfates neutralised with one or more cationic moieties;
   (3) from 5% to 80% by weight of a water-soluble inorganic salt selected from the group consisting of sodium and potassium sulfates, chlorïdes, carbonates, phosphates and mixtures thereof,
   in which the composition also includes 5 to 60% by weight of an alkylpolyglucoside surfactant of the formula $R^2O(C_NH_{2n}O)_tZ_x$ where Z is derived from glucose, $R^2$ is an alkyl group containing 12 to 18 carbon atoms, n is 2 or 3 t is 0 to 10, and x is from 1.5 to 4, and including less than 10% unreached fatty alcohol and less than 50% short chain alkylpolyglucoside an in which the composition has been made by agglomeration.

2. A composition according to claim 1 in which x is 1.6 to 2.7.

3. A composition according to any preceding claim wherein the cationic moieties for the cosurfactants (1) and (2) are selected from the group consisting of sodium potassium, ammonium, monoethanolammonium, diethanolammonium triethanolammonium, magnesium and mixtures thereof.

4. A composition of claim 3 wherein the phenyl portion of the alkylbenzene sulfonate is attached near the middle of the alkyl chain and the cationic moiety is magnesium.

## Patentansprüche

1. Eine grenzflächenaktive Verbindungen enthaltende, körnige Feinwaschzusammensetzung enthaltend:
   (1) 5 Gew.-% bis 60 Gew.-% eines Alkylbenzolsulfonat-cogrenzflächenaktiven Mittels, in welchem die Alkylgruppe 10 bis 13 Kohlenstoffatome enthält, wobei das genannte Alkylbenzolsulfonat mit einem oder mehreren kationischen Resten neutralisiert ist;
   (2) 5 Gew.-% bis 60 Gew.-% eines

Alkylpolyethoxylatsulfat-cogrenzflächenaktiven Mittels, in welchem die Alkylgruppe 10 bis 16 Kohlenstoffatome enthält, und 1 bis 6 Ethoxylatgruppen vorliegen, wobei die genannten Alkylpolyethoxylatsulfate mit einem oder mehreren kationischen Resten neutralisiert sind; und

(3) 5 Gew.-% bis 80 Gew.-% eines wasserlöslichen, anorganischen Salzes, ausgewählt aus der Gruppe, die aus Natrium- und Kaliumsulfaten, -chloriden, -carbonaten, -phosphaten, und Mischungen davon, besteht,

wobei die Zusammensetzung auch 5 Gew.-% bis 60 Gew.-% eines Alkylpolyglucosidgrenzflächenaktiven Mittels der Formel $R^2O(C_nH_{2n}O)_tZ_x$ enthält, worin sich Z aus Glucose herleitet, $R^2$ eine Alkylgruppe mit 12 bis 18 Kohlenstoffatomen darstellt, n 2 oder 3 ist, t von 0 bis 10 beträgt, und x von 1,5 bis 4 ist, und welches weniger als 10 % an nicht-umgesetztem Fettalkohol und weniger als 50 % an kurzkettigem Alkylpolyglucosid enthält, und worin die Zusammensetzung durch Agglomerieren hergestellt wurde.

2. Eine Zusammensetzung nach Anspruch 1, wobei x von 1,6 bis 2,7 beträgt.

3. Eine Zusammensetzung nach einem der vorstehenden Ansprüche, wobei die kationischen Reste für die cogrenzflächenaktiven Mittel (1) und (2) aus der Gruppe ausgewählt sind, die aus Natrium, Kalium, Ammonium, Monoethanolammonium, Diethanolammonium, Triethanolammonium, Magnesium, und Mischungen davon, besteht.

4. Eine Zusammensetzung nach Anspruch 3, wobei der Phenylabschnitt des Alkylbenzolsulfonates in der Nähe der Mitte der Alkylkette gebunden ist, und der kationische Rest Magnesium ist.

**Revendications**

1. Composition granulée détergente pour lavages délicats comprenant

(1) 5 % à 60 % en poids d'un co-agent tensioactif à base d'alkyl benzène sulfonate dans lequel le groupe alkyle contient 10 à 13 atomes de carbone, cet alkyl benzène sulfonate étant neutralisé par un ou plusieurs groupes cationiques;

(2) 5 % à 60 % en poids d'un co-agent tensioactif à base d'alkylpolyéthoxylate sulfate dans lequel le groupe alkyle contient 10 à 16 atomes de carbone et où il y a 1 à 6 groupes éthoxylate, les alkylpolyéthoxylate sulfates étant neutralisés par un ou plusieurs groupes cationiques;

(3) 5 à 80 % en poids d'un sel minéral soluble dans l'eau choisi parmi le groupe constitué des sulfates, chlorures, carbonates, phosphates de sodium et de potassium et de leurs mélanges,

la composition contenant également 5 à 60 % en poids d'un agent tensio-actif à base d'alkylpolyglucoside de formule $R^2O(C_nH_{2n}O)_tZ_n$ dans laquelle Z est dérivé du glucose, $R^2$ est un groupe alkyle contenant 12 à 18 atomes de carbone, n est égal à 2 ou 3, t est égal à 0 à 10 et x est un nombre de 1,5 à 4, et comprenant moins de 10 % d'alcool gras n'ayant pas réagi et moins de 50 % d'alkylpolyglucoside à chaîne courte, la composition ayant été préparée par agglomération.

2. Composition selon la revendication 1, dans laquelle x est un nombre de 1,6 à 2,7.

3. Composition selon l'une quelconque des revendications précédentes, dans laquelle les groupes cationiques pour les co-agents tensio-actifs (1) et (2) sont choisis parmi le groupe constitué du sodium, du potassium, de l'ammonium, du monoéthanolammonium, du diéthanolammonium, du triéthanolammonium, du magnésium et de leurs mélanges.

4. Composition selon la revendication 3, dans laquelle la partie phényle de l'alkylbenzène sulfonate est fixée à proximité du centre de la chaîne alkyle et le groupe cationique est le magnésium.